# EUROPEAN PATENT APPLICATION

(11) **EP 4 360 755 A1**
(43) Date of publication of application: **01.05.2024**
(21) Application number: 21951980.8
(22) Date of filing: 28.07.2021
(51) Int. Cl.: B01J 23/89, B01J 23/63, B01J 23/46, B01J 23/42, B01J 37/02, C07D 307/46

(54) **CATALYST FOR MAKING DICARBOXYL ACID AROMATIC HETEROCYCLIC COMPOUND, AND METHOD FOR PREPARING DICARBOXYL ACID AROMATIC HETEROCYCLIC COMPOUND**

(71) Applicant: Kolon Industries, Inc., Seoul 07793 (KR)
(72) Inventor: JEONG, Mee Hye, Seoul 07793 (KR); KIM, Jun Yeong, Seoul 07793 (KR); YONG, Da Kyoung, Seoul 07793 (KR); HA, Ji Min, Seoul 07793 (KR); PARK, Ki Hyun, Seoul 07793 (KR)
(74) Representative: Ter Meer Steinmeister & Partner
(86) International application number: PCT/KR2021/009829
(87) International publication number: WO 2023/008610

(57) **Abstract**

The present invention relates to a heterogeneous catalyst for making a dicarboxyl acid aromatic heterocyclic compound and a method for preparing a dicarboxyl acid aromatic heterocyclic compound, and according to the present invention, an oxide with improved yield and purity can be produced by an oxidation reaction of a bio-based aromatic heterocyclic compound under a heterogeneous catalyst.

## Description

### [Technical Field]

The present invention relates to a catalyst for making dicarboxyl acid aromatic heterocyclic compounds from novel raw materials and a method for preparing dicarboxyl acid aromatic heterocyclic compounds.

### [Background Art]

In order to reduce greenhouse gases, developed countries such as the United States and Europe have been strengthening carbon dioxide emission regulations. Therefore, the demand for biochemical industries, which can lower dependence on existing fossil raw materials but reduce greenhouse gases are increasing. In other words, chemical industries are changing from oil-dependent to bio-dependent.

For example, technologies to replace polyethylene terephthalate (PET), which is mainly used in beverage bottles and food storage containers, with bio-PET are being actively researched.

Dicarboxyl acid aromatic heterocyclic compounds useful as monomers in the production of bio-PET are derivatives usually prepared by oxidizing 5-hydroxymethylfurfural. Since the 5-hydroxymethylfurfural (HMF) is obtained from sugar, it is a derivative of a raw material widely available in nature.

For reference, in an oxidation reaction for 5-HMF, as shown in the following reaction scheme, 2,5-furane dianionic crosslinking agent (FDCA), 2,4-furane dianionic crosslinking agent, and an aromatic heterocyclic compound of monocarboxyl acid or dicarboxyl acid including 2-carboxy-5-formyl furan (FFCA) can be obtained as the main product.

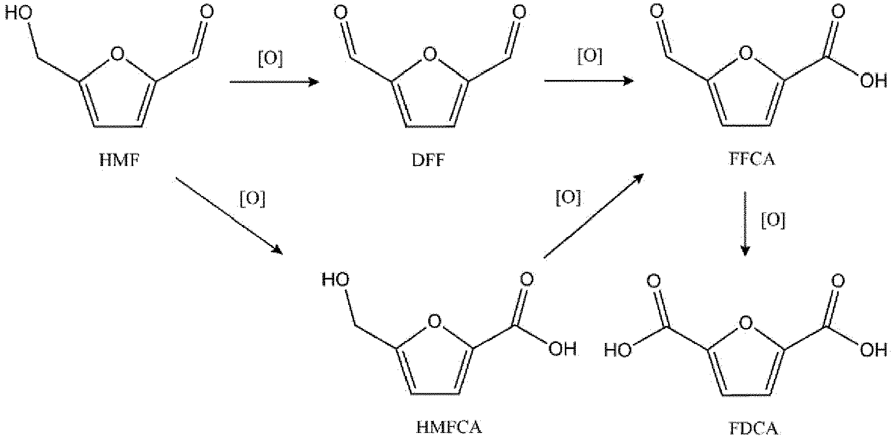

Processes for oxidation of HMF by which aromatic heterocyclic compounds of the monocarboxyl acids or dicarboxyl acids can be obtained as main products are known in the literature. For example, US 4,977,283 (Hoechst) describes a process for the oxidation of HMF carried out by a metal catalyst belonging to the platinum group in an aqueous environment at pH 6.5 to 8. The patent discloses that the ratio between various oxidation products and by-products can be adjusted by adjusting pH. According to the information in the patent, control of pH can be achieved by maintaining the pH usually below 8 through bases such as sodium or potassium hydroxide, acids or buffer solutions.

Additionally, patent application US 2008/0103318 (Battelle) describes a method for oxidation of HMF promoted by platinum supported on a carrier.

However, the above methods of oxidation of HMF have high manufacturing costs and are performed as a batch reaction. This batch reaction requires time that is substantially unrelated to the reaction, such as inputting raw materials, heating to the reaction temperature, and discharging reaction products, and has the problem of lowering the productivity of the reactor. In addition, in order to use the catalyst repeatedly, a separation process between the catalyst and the product is required, and there is also a problem of early deterioration of the catalyst's activity due to changes in temperature, etc.

### [Prior art documents]

### [Patent documents]

U.S. Patent US 4,977,283

### [Disclosure]

### [Description of the Drawings]

### [Technical Problem]

One aspect of the present invention is to provide a heterogeneous catalyst used in the synthesis of dicarboxyl acid aromatic heterocyclic compounds using novel raw materials.

Another aspect of the present invention is to provide a method for preparing the aforementioned heterogeneous catalyst.

Another aspect of the present invention is to provide a method for preparing a dicarboxyl acid aromatic heterocyclic compound that is environmentally friendly and has improved production efficiency by using bio-based raw materials as the aromatic heterocyclic compound raw material.

### [Technical Solution]

According to the first aspect of the present invention, as a catalyst for preparing dicarboxyl acid aromatic heterocyclic compound, a heterogeneous catalyst having a structure in which cations of a metal catalyst component are bonded to a porous central metal-organic framework material is provided.

Additionally, the metal catalyst component may be one or more selected from Fe, Ni, Ru, Rh, Pd, Os, Ir, Au, and Pt.

Additionally, the porous central metal may be one or more types selected from a skeleton support material, an active metal, and an activity-promoting metal.

Additionally, the organic framework material may be an anion provided from an anionic crosslinking agent.

Additionally, the anionic crosslinking agent may be one or more polyanionic substances selected from citric acid, tartaric acid, malic acid, malonic acid, and tartaric acid.

Additionally, the heterogeneous catalyst may produce a dicarboxyl acid aromatic heterocyclic compound by oxidizing an aromatic heterocyclic raw material having a structure represented by Chemical Formula 1: 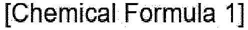 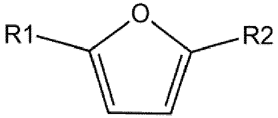 (wherein, in above chemical formula, any one of R1 and R2 is an aldehyde and the other is a substituted or unsubstituted (C1-C20) alkyl, acetoxy, or aldehyde, the substituted (C1-C20) alkyl is substituted with one or more functional groups selected from bromine, chlorine, fluorine, and iodine).

In addition, the heterogeneous catalyst may be prepared by oxidizing one or more compounds selected from a compound having a structure represented by Chemical Formula 2, a compound having a structure represented by Chemical Formula 3, and a compound having a structure represented by Chemical Formula 4 in a polar solvent to obtain an oxide having the structure represented by Chemical Formula 5:

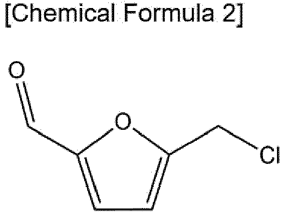

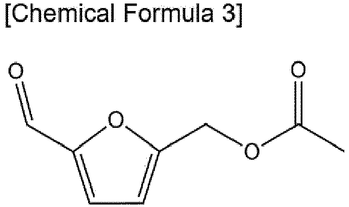

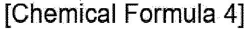

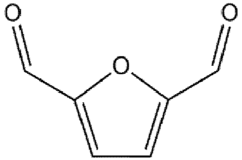

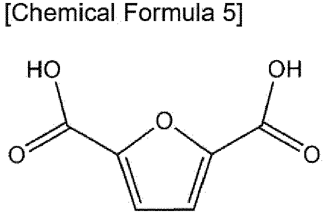

The heterogeneous catalyst may be one or more selected from Ru/MnCo₂O₄, Au/CeO₂, Ru/C and Pt/C at a molar ratio of 0.4 to 1.0 based on 1 mole of the aromatic heterocyclic compound having the structure represented by Chemical Formula 1.

According to a second aspect of the present invention, a method for preparing a heterogeneous catalyst includes preparing a solution including a porous central metal oxide; preparing a porous central metal-organic framework material by reacting the porous central metal oxide solution with an anionic crosslinking agent; and impregnating the porous central metal-organic framework material with a precursor solution of the metal catalyst component to provide a structure in which cations of the metal catalyst component are bonded.

In addition, the second step may include a 2-1 step of mixing the porous central metal oxide solution and an anionic crosslinking agent; a 2-2 step of heating the mixed solution after the step 2-1 to a first temperature and then performing a primary reaction of the mixed solution; and a 2-3 step of heating the mixed solution after the step 2-2 to a second temperature higher than the first temperature and then subjecting the mixed solution to a secondary reaction.

In addition, the third step may include a 3-1 step of impregnating the reactant after the second step with a precursor solution of the metal catalyst component obtained by dissolving the precursor of the metal catalyst component in a solvent; and a 3-2 step of firing the impregnated product after step 3-1 at a third temperature lower than the second temperature.

Additionally, the solvent used to prepare the porous central metal oxide solution and the precursor solution of the metal catalyst component may be water, alcohol, or a combination thereof.

Additionally, the anionic crosslinking agent may be citric acid, tartaric acid, malic acid, malonic acid, tartaric acid, and a combination thereof.

Additionally, the first temperature may be in the range of 40 to 200 °C, the second temperature may be in the range of 400 to 1000 °C, and the step 2-3 may be performed for 3 to 12 hours.

Additionally, the step 3-1 may be performed in the range of 40 to 80 °C, and the third temperature may be in the range of 40 to 800 °C.

According to a third aspect of the present invention, a method for preparing a dicarboxyl acid aromatic heterocyclic compound includes oxidizing an aromatic heterocyclic compound raw material in a polar solvent in the presence of a heterogeneous catalyst to prepare dicarboxyl acid aromatic heterocyclic compounds wherein the heterogeneous catalyst is the aforementioned catalyst, and the aromatic heterocyclic compound raw material is a hydroxyl-free compound having a structure represented by Chemical Formula 1: 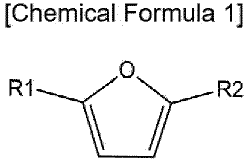 (In the above chemical formula, any one of R1 and R2 is an aldehyde, and the others are substituted or unsubstituted (C1 to C20) alkyl, acetoxy, or aldehyde, and the substituted (C1 to C20) alkyl is substituted with one or more functional groups selected from bromine, chlorine, fluorine and iodine).

In addition, the aromatic heterocyclic compound having the structure represented by Chemical Formula 1 may be used in a state modified to the structure represented by Chemical Formula 3 or the structure represented by Chemical Formula 4:

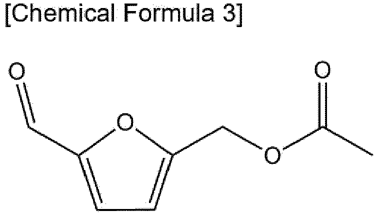

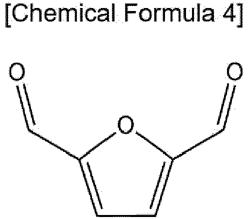

In addition, the oxidation reaction may be performed by a catalyst-free oxidation step of oxidizing an aromatic heterocyclic compound having a structure represented by Chemical Formula 2 at a fourth temperature in a polar solvent in the presence of a halogen-based ammonium compound and an ion activator to produce an aromatic heterocyclic compound having a structure represented by Chemical Formula 3; and an oxidation step of oxidizing an aromatic heterocyclic compound having the structure represented by Chemical Formula 3 in a polar solvent in the presence of a basic material and a heterogeneous catalyst at a fifth temperature higher than the fourth temperature to produce a dicarboxyl acid aromatic heterocyclic compound.

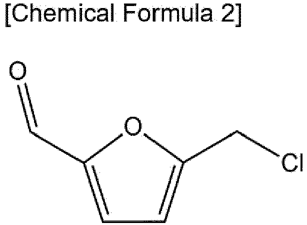

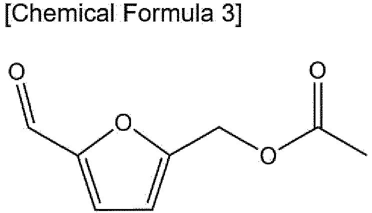

In addition, the oxidation reaction may be performed by a first catalyst-free oxidation step of oxidizing an aromatic heterocyclic compound raw material having a structure represented by Chemical Formula 2 at a sixth temperature in a polar solvent; a secondary catalyst-free oxidation step of substituting the polar solvent with another type of polar solvent and performing an oxidation reaction at a seventh temperature lower than the sixth temperature to produce an aromatic heterocyclic compound having a structure represented by Chemical Formula 4; and an oxidation step of oxidizing an aromatic heterocyclic compound having the structure represented by Chemical Formula 4 in a polar solvent at an eighth temperature lower than the sixth temperature in the presence of a heterogeneous catalyst to produce a dicarboxyl acid aromatic heterocyclic compound.

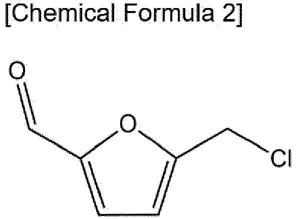

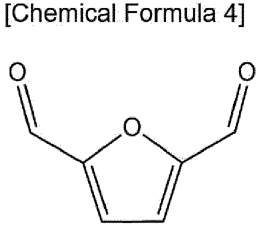

### [Advantageous Effects]

The new bio-based raw material according to one aspect of the present invention is not only environmentally friendly in that it is a material converted from wood, but is also suitable for use in the oxidation reaction of aromatic heterocyclic compounds by replacing the conventionally used 5-HMF.

The dicarboxyl acid aromatic heterocyclic compound according to another aspect of the present invention can be provided in a high yield of 95% or more in a crude composition by continuing the synthesis reaction of raw materials.

Furthermore, the method for preparing dicarboxyl acid aromatic heterocyclic compound according to another aspect of the present invention overcomes the disadvantages of the conventional method for batch synthesis using 5-HMF as a raw material by continuously synthesizing the product with high yield and has an effect of improving energy efficiency and productivity without the pressure control process within the reactor.

### [Mode for Invention]

Hereinafter, the method for preparing a dicarboxyl acid aromatic heterocyclic compound using the aromatic heterocyclic compound of the present invention as a raw material and the heterogeneous catalyst used therein will be described in detail.

Unless explicitly described to the contrary, the word "comprise," and variations such as "comprises" or "comprising," will be understood to imply the inclusion of stated elements but not the exclusion of any other elements.

In the present invention, "metal precursor" refers to a chemical substance prepared in advance to react with a metal. For example, "transition metal precursor" is a concept that refers to a chemical substance that is prepared in advance to react a transition metal.

### <First aspect of the present invention>

The first aspect of the present invention is a raw material for preparing a dicarboxyl acid aromatic heterocyclic compound, which is a hydroxyl-free aromatic heterocyclic compound including an aromatic heterocycle and a hydroxyl-free terminal end.

In the present invention, the hydroxyl-free aromatic heterocyclic compound may have a structure represented by Chemical Formula 1 : 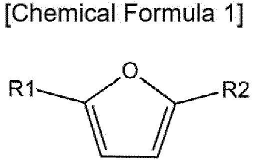 (In the above chemical formula, any one of R1 and R2 is an aldehyde, and the others are substituted or unsubstituted (C1 to C20) alkyl, acetoxy, or aldehyde, and the substituted (C1 to C20) alkyl is substituted with one or more functional groups selected from bromine, chlorine, fluorine and iodine).

The compound of Chemical Formula 1 is a compound converted from wood and is the first compound used in the preparation of the dicarboxyl acid aromatic heterocyclic compound. Unless otherwise specified herein, halogen refers to chlorine, bromine, fluorine, and iodine.

In the present invention, aromatic heterocycle refers to a structure that provides a skeleton when preparing the dicarboxyl acid aromatic heterocyclic compound.

For example, the aromatic heterocycle may be one or more selected from furan, thiophene, and pyrrole.

In addition, the hydroxyl-free terminal end may be one or more selected from an aldehyde group, an acetoxy group, and an alkyl group having 1 to 20 carbon atoms, where the alkyl group may be unsubstituted or halogen-substituted.

In Chemical Formula 1, when R1 is an aldehyde, R2 is selected from hydrogen, aldehyde, acetoxy, halogen, C1-C20 alkyl, and C1-C20 alkoxy, wherein C1-C20 alkyl or C1-C20 alkoxy may be unsubstituted or substituted with the above halogen atom.

Additionally, in Chemical Formula 1, when R1 is an aldehyde, R2 is selected from acetoxy or C1-C20 alkyl, where the C1-C20 alkyl may be substituted with one or more halogen atoms.

At this time, the hydroxyl-free aromatic heterocyclic compound may have one or more structures selected from Chemical Formulas 2 to 4.

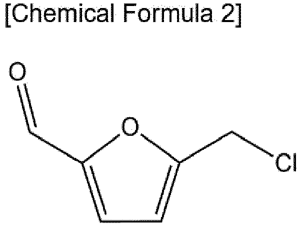

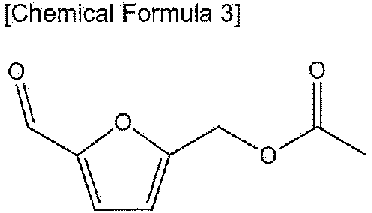

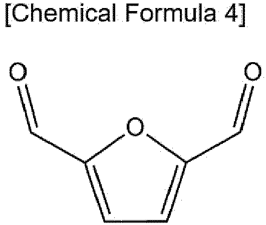

### <Second aspect of the present invention>

The second aspect of the present invention relates to a catalyst for preparing dicarboxyl acid aromatic heterocyclic compound, which is a heterogeneous catalyst having a structure in which cations of a metal catalyst component are bonded to a porous central metal-organic framework material. In the present invention, the porous central metal-organic framework material refers to a porous organic-inorganic compound formed by combining a central metal ion with an organic ligand, and may include organic and inorganic substances within the skeletal structure or on the surface and may include a crystalline compound having a molecular- or nano-sized pore structure.

In addition, in the present invention, the metal catalyst component refers to a component that has an activity that contributes to the improvement of product yield by using the raw materials of the first aspect described above when preparing the dicarboxyl acid aromatic heterocyclic compound and is inserted into the pore structure of the framework material.

The metal catalyst may be at least one selected from Fe, Ni, Ru, Rh, Pd, Os, Ir, Au, and Pt, which may have various oxidation numbers, and Ru, Pd, Pt, and Au are desirably used.

In addition, the central metal may be selected from one or more of skeletal support materials such as Mg and Al, active metals such as Mn and Co, and activity-promoting metals such as Ce, depending on the activity of the reaction target.

For example, two types of active metals, Mn and Co, can be selected and used as the central metal, or Ce, an activity-promoting metal known to have excellent storage and transport capabilities for reactive oxygen species, can be used alone as a central metal.

As a specific example, the central metal framework material used in the present invention may include a structure represented by Chemical Formula 9.

[Chemical Formula 8] (Mn)ₐ-(Co)_{b}-(Ce)_{c}-O_{d}

(In the above chemical formula, a, b, and c represent oxidation numbers, a sum of a, b, and c is greater than 1, a sum of a and c is greater than 0, and d is an integer that is 1 greater than a+b+c)

The central metal framework material having this structure has a binding site in the pore structure, and can provide a functionalized heterogeneous catalyst when a metal catalyst component containing an element capable of binding to the binding site in the pore structure is combined.

At this time, the organic framework material may be provided using an anionic crosslinking agent. In the present invention, an anionic crosslinking agent is a compound that provides anions to serve as a crosslinking agent between the porous central metal and the metal catalyst component that is subsequently combined, thereby providing a form in which the cations of the metal catalyst component are strongly adsorbed on the particles of the porous central metal.

In the case of aliphatic substances without a ring structure, anionic crosslinking agents volatilize before the particles are agglomerated, creating pores and since they act as pore-inducing substances themselves, no separate surfactant is needed, and after the particles are agglomerated, ligands can be provided to induce modification on the particle surface and induce subsequent adsorption bonding.

The anionic crosslinking agent may be a substance that provides one or more polyvalent anions selected from citric acid, tartaric acid, malic acid, malonic acid, and tartaric acid.

In the present invention, the heterogeneous catalyst may use the above-described porous central metal as a carrier or materials known in the art such as carbon.

At this time, the shape of the carrier may vary. It is not limited to spherical or cylindrical shapes with micropores.

The catalyst may be Ru-MnCO₂O₄, Au-CeO₂, etc., and Pt/C, Pd/C, Rh/C, etc., which are known as heterogeneous catalysts in the art, may be used in combination or replacement.

An amount of catalyst used is not particularly limited, but for example, a molar ratio of 600 mol or less, or 50 to 600 mol, based on the central metal and metal catalyst component in the catalyst, based on 1 mole of the aromatic heterocyclic compound raw material having the structure represented by Chemical Formula 1.

The heterogeneous catalyst according to the present invention overcomes the disadvantage of melting in the reaction solution and not being easy to separate when using a homogeneous catalyst, and provides the advantage of being able to be recycled multiple times after separation, and increases the yield when preparing the dicarboxyl acid aromatic heterocyclic compound.

The catalyst can be used in the oxidation reaction described later by circulating it more than once, for example, more than 4 times.

In addition, the heterogeneous catalyst may produce the dicarboxyl acid aromatic heterocyclic compound by oxidizing an aromatic heterocyclic raw material having a structure represented by Chemical Formula 1: 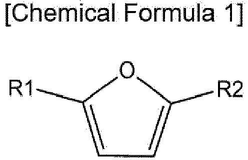 (wherein, in above chemical formula, any one of R1 and R2 is an aldehyde and the other is a substituted or unsubstituted (C1-C20) alkyl, acetoxy, or aldehyde, the substituted (C1-C20) alkyl is substituted with one or more functional groups selected from bromine, chlorine, fluorine, and iodine).

In addition, the heterogeneous catalyst is prepared by oxidizing at least one compound selected from a compound having a structure represented by Chemical Formula 2, a compound having a structure represented by Chemical Formula 3, and a compound having a structure represented Chemical Formula 4 in a polar solvent to produce an oxide having the structure indicated by Chemical Formula 5:

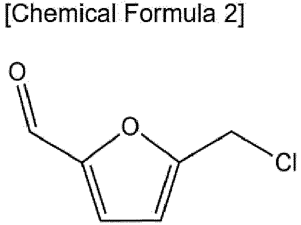

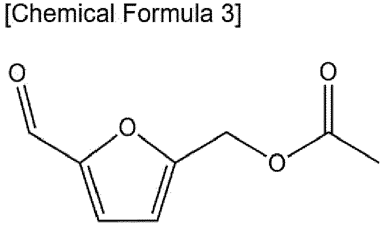

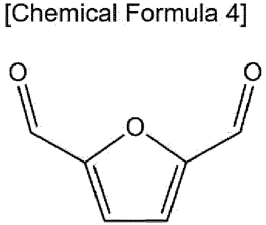

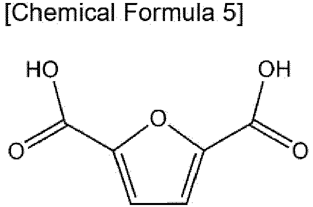

Additionally, the dicarboxyl acid aromatic heterocyclic compound may have a structure represented by Chemical Formula 5:

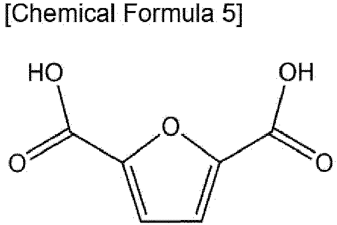

In addition, the heterogeneous catalyst is one or more selected from Ru/MnCo₂O₄, Au/CeO₂, Ru/C, Pt/C at a molar ratio of 0.4 to 1.0 based on 1 mole of the aromatic heterocyclic compound having the structure represented by Chemical Formula 1.

### <Third aspect of the present invention>

A third aspect of the present invention is a method for preparing the aforementioned heterogeneous catalyst.

The heterogeneous catalyst is first prepared as a porous central metal-organic framework material by reacting a solution including a porous central metal oxide with an anionic crosslinking agent. It is then prepared by impregnating the porous central metal-organic framework material into a metal catalyst precursor and then thermally decomposing it. Specifically, the preparing method includes a first step, a second step, and a third step. The name of each step is a name given to distinguish each step from other steps and does not include all technical meanings of each step.

The first step is a step of preparing a solution including the porous central metal oxide (hereinafter also referred to as a porous central metal precursor).

The porous central metal oxide may be may be one or more selected from, for example, dimethyl manganese, diethyl manganese, manganese acetate, manganese acetate dihydrate, manganese acetylacetonate, manganese acetylacetonate hydrate, manganese iodide, manganese bromide, manganese chloride, manganese fluoride, manganese fluoride tetrahydrate, manganese carbonate, manganese cyanide, manganese nitrate, manganese nitrate hexahydrate, manganese oxide, manganese peroxide, manganese perchlorate, manganese perchlorate hexahydrate, manganese sulfate, diphenyl manganese, manganese naphthalate, manganese oleate, and manganese stearate, when the transition metal is manganese.

Accordingly, the same can be applied even when the porous central metal is not manganese.

The aforementioned porous central metal may be provided in a form having a pore structure of molecular size or nano size and may be provided as a support, that is, a carrier.

The porous central metal may be one or more types selected from Mn, Co, Mg, and Ce.

The second step is a step of reacting the porous central metal oxide solution of the first step with an anion crosslinking agent to prepare a porous central metal-organic framework material.

An anionic crosslinking agent is necessary to provide anions to act as a crosslinking agent between the porous central metal and the subsequently combined metal catalyst component, thereby providing a form in which cations of the metal catalyst component are adsorbed on the particles of the porous central metal and citric acid, tartaric acid, malic acid, malonic acid, tartaric acid, etc. may be used.

Specifically, the second step includes step 2-1, step 2-2, and step 2-3.

The 2-1 step is a step of mixing a porous central metal oxide solution and an anionic crosslinking agent. The porous central metal oxide can be used in solution form using water, alcohol, and a mixture thereof.

In addition, the porous central metal oxide and the anionic crosslinking agent can be mixed at a molar ratio of, for example, 1:1 to 1:5 to prepare a mixed solution.

The step 2-2 is a step of heating the mixed solution after the step 2-1 to the first temperature and then subjecting the mixed solution to a primary reaction. The range of the first temperature may be, for example, 40 °C to 200 °C, and most desirably 100 °C to 180 °C. At this time, the pressure remains the same.

The step 2-3 is a step in which the mixed solution after the step 2-2 is heated to a second temperature higher than the first temperature and then subjected to a secondary reaction of the mixed solution. The second temperature may range from 400 °C to 1000 °C, and is desirably higher than the first temperature. At this time, the pressure remains the same. Additionally, the reaction time at this time is, for example, 2 to 24 hours, and desirably 3 to 12 hours.

The third step is a step of impregnating the porous central metal-organic framework material prepared after the second step (specifically, the step 2-3) into a precursor solution of the metal catalyst component to form a structure in which cations of the metal catalyst component are bonded.

Specifically, the third step includes step 3-1 and step 3-2.

The 3-1 step is a step of impregnating the reactant after the second step (specifically, the step 2-3) with a solution of the precursor of the metal catalyst component obtained by dissolving the precursor of the metal catalyst component in a solvent. The step 3-1 can be performed in the range of 40 °C to 80 °C, and the pressure can be maintained at normal pressure.

The 3-2 step is a step of baking the impregnated product after the 3-1 step at a third temperature lower than the second temperature. The third temperature may range from 40 to 800 °C or 100°C to 600 °C, and is desirably higher than the first temperature. At this time, the pressure remains the same.

The solvent used to prepare the porous central metal oxide solution of the step 2-1 and the precursor solution of the metal catalyst component of the step 3-1 is used to control a concentration of the mixed solution, and may include water, alcohol, and a combination thereof.

### <Fourth aspect of the present invention>

The method for preparing a dicarboxyl acid aromatic compound prepares a dicarboxyl acid aromatic heterocyclic compound by subjecting the above-described aromatic heterocyclic compound raw material to an oxidation reaction in a polar solvent in the presence of a heterogeneous catalyst. Since the raw materials have been described in detail in the first aspect described above, detailed descriptions of repeated descriptions will be omitted.

As described above, the raw material of the present invention may be an aromatic heterocyclic compound having a structure represented by Chemical Formula 1, and for example, it may be an aromatic heterocyclic compound having a structure represented by Chemical Formula 2.

The aromatic heterocyclic compound having the structure represented by Chemical Formula 2 may be used in a modified state to have a structure represented by Chemical Formula 3 or a structure represented by Chemical Formula 4.

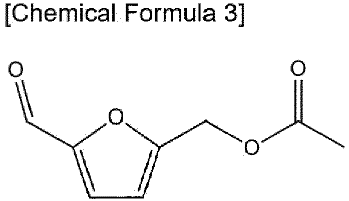

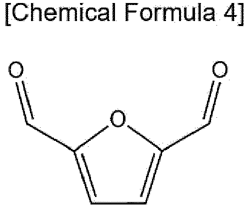

In order to increase reaction efficiency in an oxidation reaction, the conversion rate should be maximized and the molar ratio of the two reacting functional groups must be close to 1:1. Maximizing conversion rate can be achieved by lengthening the reaction time or increasing the reaction temperature, but controlling the molar number of the two reacting functional groups to be 1:1 is not easy, which often acts as a limiting factor in preparing a specific dicarboxyl acid aromatic compound (for example, a dicarboxyl acid aromatic compound represented by Chemical Formula 5) in high yield in an oxidation reaction. For this reason, when preparing a dicarboxyl acid aromatic compound having a structure represented by Chemical Formula 5, it is desirable to produce high yield dicarboxyl acid aromatic compounds in two steps as follows rather than preparing a dicarboxyl acid heteroaromatic compound having a structure represented by Chemical Formula 2 by oxidation. That is, first, a catalyst-free oxidation reaction is performed prior to the catalytic oxidation reaction to obtain a compound in which one terminal end is oxidized, and in the second step, an oxidation reaction is performed under a catalyst to oxidize the remaining terminal end.

As a specific example, the oxidation reaction according to the present invention can be expressed in the following two cases depending on the modification structure.

For example, when the oxidation reaction includes a catalyst-free oxidation step and an oxidation step, the name of each step is a name given to distinguish each step from other steps and does not include all technical meanings of each step.

The catalyst-free oxidation step is a step of preparing an aromatic heterocyclic compound having the structure represented by Chemical Formula 3.

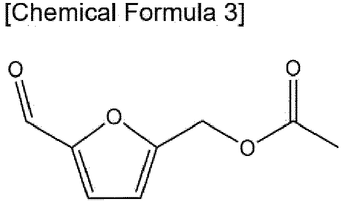

The aromatic heterocyclic compound having the structure is prepared by oxidizing an aromatic heterocyclic compound raw material having the structure represented by Chemical Formula 2 in a polar solvent in the presence of an onium compound and an ion activator at the fourth temperature. The fourth temperature may be 10 °C to 80 °C or 30 °C to 50 °C. The pressure may be normal pressure, and the reaction may be performed for 1 hr to 10 hr, or 2 hr to 6 hr.

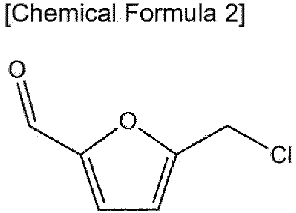

The onium-based compound used in the present invention is a compound composed of an onium-based cation and an anion with a pKa value of 3 or less. The onium-based cation is broadly defined by IUPAC, and representative examples include tetraalkylammonium and tetraalkyl having an alkyl group of 4 to 10 carbon atoms, (or aryl) phosphonium cation, etc.

There are many anions with a pKa value of 3 or less, but among them, halide anions are preferable as they are easily formed through a neutralization reaction with onium-based cations.

The onium-based compounds may be at least one selected from, for example, tetrabutylammonium chloride (TBAC), tetrahexylammonium chloride (THAC), tetraoctylammonium chloride (TOAC), tetrabutylammonium bromide (TBAB), and tetrabutylammonium iodide (TBAI).

The onium-based compound used in the present invention can be used in an amount of 0.4 to 1.0 mole based on 1 mole of the aromatic heterocyclic compound represented by Chemical Formula 2.

In addition, the ion activator may be, for example, one or more selected from NaOAC and KOAc, and may be used in a molar ratio of 1 to 5 based on 1 mole of the aromatic heterocyclic compound represented by Chemical Formula 2.

In addition, the polar solvent may be, for example, one or more selected from acetonitrile, acetone, dimethylformamide, dimethyl sulfoxide, water, ethanol, methanol, and t-butyl hypochlorite (t-BuOCI), and may be used in a molar ratio of 1 to 5 based on 1 mole of the aromatic heterocyclic compound represented by Chemical Formula 2.

The oxidation step is a step of preparing a desired dicarboxyl acid aromatic heterocyclic compound from the aromatic heterocyclic compound having the structure represented by Chemical Formula 3.

The desired compound is prepared by oxidizing a basic material in a polar solvent under the presence of a heterogeneous catalyst at a fifth temperature higher than the fourth temperature. The fifth temperature may be 80 °C to 200 °C and is preferable to be within a higher range than the fourth temperature. In addition, the corresponding reaction may be performed under a normal pressure condition and for 2 to 10 hours.

The basic compound used in the present invention may be for example selected from salts of sodium, lithium, calcium, magnesium, or ammonium, which are substituted with 1 to 4 alkyl groups having 1 to 15 carbon atoms, or even from monoethanol amine, diethanol amine, triethanol amine, aminoethyl propanediol, methyl glucamine, and a mixture thereof.

Specific examples of the basic compound may be at least one selected from sodium bicarbonate, potassium bicarbonate, sodium carbonate, potassium carbonate, calcium carbonate, magnesium carbonate, calcium hydroxide, magnesium hydroxide, dibasic and tribasic phosphate buffer solutions, and the like.

The basic compound is a weak base of which pH is in a range of greater than 7 and less than or equal to 11 and specifically, may be continuously added to the reaction solution or intermittently added thereto before, during, or after the reaction.

The heterogeneous catalyst may be, for example, at least one selected from Ru-MnCo₂O₄, Au-CeO₂, and Pt/C, but considering product-manufacturing efficiency and at least once or at least 4 times recycling, the Ru-MnCo₂O₄ may be desirably used.

Herein, the polar solvent may be a known polar solvent, for example, dimethylsulfoxide, ethanol, acetonitrile, and the like. The polar solvent may be used within a range of 10 to 100 wt% based on a weight of the aromatic heterocyclic compound with the structure represented by Chemical Formula 3, but is not limited thereto.

For another example, in the oxidation reaction including a primary catalyst-free oxidation step, a secondary catalyst-free oxidation step, and an oxidation step, each step is named to be distinguished from the other steps but not contain all the technical meanings of each own step.

The primary catalyst-free oxidation step is a step of producing a portion of the aromatic heterocyclic compound having the structure represented by Chemical Formula 4.

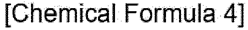

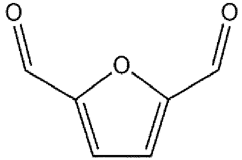

The aromatic heterocyclic compound having the corresponding structure is prepared by oxidizing an aromatic heterocyclic compound raw material having the structure represented by Chemical Formula 2 in a polar solvent at a sixth temperature. The sixth temperature may be 100 °C to 200 °C. The reaction may be performed under a normal pressure for 8 hr to 24 hr.

Herein, the polar solvent may be a known polar solvent, for example, dimethylsulfoxide, ethanol, acetonitrile, and the like. The polar solvent may be used within a range of 10 to 100 wt% based on a weight of the aromatic heterocyclic compound with the structure represented by Chemical Formula 2.

The secondary catalyst-free oxidation step is a step of producing the aromatic heterocyclic compound with the structure represented by Chemical Formula 4 at maximum.

The aromatic heterocyclic compound with the corresponding structure may be prepared by substituting the polar solvent used in the primary catalyst-free oxidation step with different types of polar solvents and additionally, performing an oxidation reaction at a lower seventh temperature than the sixth temperature.

Herein, the substituted polar solvent may be a known polar solvent, for example, t-BuOCI with improved solubility compared with the solvent used before the substitution. The corresponding polar solvent may be used within a range of 10 to 100 wt% based on the weight of aromatic heterocyclic compound having the structure represented by Chemical Formula 2 but is not limited thereto.

The seventh temperature may be within a range of 10 °C to 80 °C and is desirable to lower from the sixth temperature. Herein, the reaction may be performed under a normal pressure for 1 hr to 10 hr.

The oxidation step is a step of producing a desired dicarboxyl acid aromatic heterocyclic compound from the aromatic heterocyclic compound with a structure represented by represented by Chemical Formula 4.

The desired compound may be prepared through an oxidation reaction in a polar solvent under the presence of a heterogeneous catalyst at an eighth temperature between the sixth temperature and the seventh temperature. The eighth temperature may be within a range of 10 °C to 80 °C and is desirable to lower from the sixth temperature. Herein, the corresponding reaction may be performed under a normal pressure for 2 hours to 15 hours.

The heterogeneous catalyst may be, for example, at least one selected from Ru-MnCo₂O₄, Au-CeO₂, Ru/C, and Pt/C and in consideration of product-manufacturing efficiency and at least once or at least 4 times recycling, the Ru-MnCo₂O₄ or the Au-CeO₂ may be desirably used.

Herein, the used polar solvent may be a known polar solvent, for example, ethanol. The corresponding polar solvent may be used within a range of 10 to 100 wt% based on the weight of the aromatic heterocyclic compound having the structure represented by Chemical Formula 4 but is not limited thereto.

### <Fifth aspect of the present invention>

A fifth aspect of the present invention is a crude composition prepared by using the aromatic heterocyclic compound raw material according to the first aspect and the heterogeneous catalyst according to the second aspect.

The composition of the fifth aspect includes a dicarboxyl acid aromatic heterocyclic compound.

The target dicarboxyl acid aromatic heterocyclic compound is a compound having a structure represented by Chemical Formula 5.

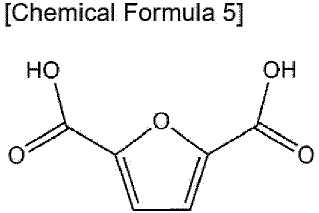

Herein, the composition inevitably includes a raw material (corresponding to the aromatic heterocyclic compound having the structure represented by Chemical Formula 2), a modifying material (the aromatic heterocyclic compound having the structure represented by Chemical Formula 3 or the aromatic heterocyclic compound having the structure represented by Chemical Formula 4), additional oxides (the aromatic heterocyclic compound having the structure represented by Chemical Formula 6 (FFCA) or the aromatic heterocyclic compound represented by the structure represented by Chemical Formula 7).

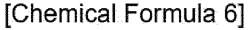

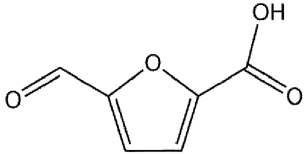

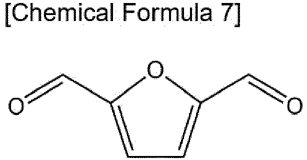

For reference, the aforementioned raw material is used for an oxidation reaction under the presence of a heterogeneous catalyst, which is a targeted reaction of the present invention and specifically, a reaction of contacting the aromatic heterocyclic compound represented by Chemical Formula 1 with an oxidizing agent (oxygen gas, etc.) under the presence of a heterogeneous catalyst to oxidize an unsaturated group included in the backbone of the aromatic heterocyclic compound.

As the unsaturated group included in the backbone of the aromatic heterocyclic compound, as shown in the following reaction scheme, is oxidized, oxides (hereinafter, referred to as dicarboxyl acid aromatic heterocyclic compounds or reaction products) of the aromatic heterocyclic compound may be obtained from the reaction and include 2,5-furandianionic crosslinking agents (corresponding to FDCA), 2,4-furandianionic crosslinking agents, 2-carboxyl-5-formyl furan (corresponding to FFCA), levulinic acid, or the like.

These products, which in general have a higher melting point than the raw material of the aromatic heterocyclic compound, are difficult to take out in a molten state from the reactor and thus require a post treatment such as purification or crystallization, etc. For example, 5-chloromethylfurfural as the aromatic heterocyclic compound has a melting point of 37 °C, but a melting point of 2,5-furandianionic crosslinking agent is 342 °C.

In addition, in order to efficiently separate these products, a dicarboxyl acid aromatic heterocyclic compound having a structure represented by Chemical Formula 5 is desirable to obtain as much as possible.

For example, the composition according to the fifth aspect of the present invention may include 95 wt% or more of the dicarboxyl acid aromatic heterocyclic compound having the structure represented by Chemical Formula 5 and the other materials in a balance amount.

### <Sixth aspect of the present invention>

The sixth aspect of the present invention is an apparatus for preparing a dicarboxyl acid aromatic heterocyclic compound by oxidizing an aromatic heterocyclic compound raw material in a polar solvent under the presence of a heterogeneous catalyst.

The apparatus according to the sixth aspect includes a reactor, a storage tank for the aromatic heterocyclic compound raw material, and a supply device of an oxidizing agent. Herein, the reactor may be a cylinder-type column reactor internally filled with a carrier supporting an active catalyst but is not limited thereto.

In addition, the supply device of the oxidizing agent is structurally connected to a tube connecting the storage tank for the aromatic heterocyclic compound raw material with the reactor and may supply the raw material in a pressurized state to the reactor.

Since the supply device for the oxidizing agent may be structurally disposed to transport the raw material in the pressurized state into the reactor, there are advantages of no need to separately or additionally control the pressurization conditions in the reactor and an increase of a reaction activation degree by the raw material.

The reactor pressure may be controlled within a range of 1 to 5 atm by the oxidizing agent supplied through the supply device for the oxidizing agent, and the reactor temperature may be controlled within a range of 40 °C to 200 °C.

The oxidizing agent may be at least one selected from oxygen and air, and the carrier may be at least one independently selected from carbon, aluminum, cerium, zirconium, and magnesium.

Herein, the aromatic heterocyclic compound raw material may have the structure represented by Chemical Formula 1.

The cylinder type column reactor may be internally filled with the aforementioned heterogeneous catalyst. The heterogeneous catalyst is the same as the above and will not be specifically described here.

Since the reactor discharges the vapor dicarboxyl acid aromatic heterocyclic compound, which is separated from a purification reactor or a crystallization reactor, at the bottom of the purification reactor or the crystallization reactor, a collection unit for the dicarboxyl acid aromatic heterocyclic compound may be included.

The reactor may include a first oxidation reactor and a second oxidation reactor, wherein the first oxidation reactor may be used as a reactor for a catalyst-free oxidation reaction, and the second oxidation reactor may be used as a reactor for a catalyst oxidation reaction.

If necessary, in the reactor, the first oxidation reactor may be used for a primary catalyst-free oxidation reaction and for a secondary catalyst-free oxidation reaction, while the second oxidation reactor may be used for the catalyst oxidation reaction.

For example, after dissolving the aromatic heterocyclic compound in the polar solvent, the solution is supplied by a pressure difference in the reactor and the storage tank for the aromatic heterocyclic compound raw material, wherein a valve may be used to control its flow rate, thereby, adjusting reaction time (retention time).

In addition, the solution passes through a column (for example, a cylinder type column) fixedly packed with the heterogeneous catalyst as a reactor and completes with a reaction and then, is transported to a post treatment reactor and stored in a collection container through the purification and/or crystallization process.

The dicarboxyl acid aromatic heterocyclic compound conversion solution stored in the collection container is discharged from the bottom to separate the solvent, the heterogeneous catalyst, and the dicarboxyl acid aromatic heterocyclic compound as the reaction products and recover the dicarboxyl acid aromatic heterocyclic compound therefrom.

According to the present invention, since the storage tank filled with the aromatic heterocyclic compound solution is pressed with the oxidizing agent (oxygen or air), oxygen may be dissolved in the solvent to improve reactivity and also, to control the reaction pressure in the reactor.

Specifically, the aromatic heterocyclic compound as a raw material, which is mixed with the solvent, is pressed by oxygen gas discharged from the oxygen supply tank or the reactor during the transport to set a concentration where a total amount of the raw material may be dissolved in the solvent including the reaction products under the pressure conditions of the reactor.

In the reactor, when the oxygen gas discharged from the reactor is used for the pressurization, a control valve, etc. may be used to control a flow rate and the like. The oxygen may be supplied from the other reactors such as the supply tank for the raw material, where the aromatic heterocyclic compound solution as the raw material is pressurized with the oxygen, a supply line, where the raw material solution is supplied to the reactor, or the like.

The aromatic heterocyclic compound as the raw material may be supplied at the top of the reactor to maximize the reaction rate but is not limited thereto.

In the reactor, the aromatic heterocyclic compound solution as the raw material may be in contact with the fixed catalyst and the oxygen, which causes an oxidation reaction, wherein a liquid mass feeder and the control valve installed at the top of the reactor may be used to all control a content of the aromatic heterocyclic compound solution raw material automatically or semiautomatically introduced into the reactor and an input amount, speed, etc. of the oxygen.

In general, an oxidation reaction rate of an aromatic heterocyclic compound is affected by an amount of oxygen gas dissolved in a reaction solution. In the method of the present invention, the aromatic heterocyclic compound raw material pressurized by oxygen is automatically or semiautomatically introduced into the reactor, which also may increase an oxygen adsorption amount of the catalyst. Accordingly, the reaction may advantageously proceed.

In addition, the storage tank of the aromatic heterocyclic compound raw material may be filled with the aromatic heterocyclic compound alone or by dissolving it in the polar solvent, for example, at a concentration of 0.5 wt% to 5.0 wt% or 0.5 wt% to 2.0 wt%.

When the aromatic heterocyclic compound alone is filled, the polar solvent may be supplied from a polar solvent supply tank to the tube connecting the storage tank of the aromatic heterocyclic compound with the reactor to meet the above concentration.

The raw material prepared as above is pressurized to 100 bar or less by the oxidizing agent (oxygen gas) supplied to the tube from the oxidizing agent storage tank. Herein, a pressure in the pressurization is proportional to an amount of oxygen dissolved in the raw material solution, wherein a higher pressure may improve the reaction rate.

As for the heterogeneous catalyst filled in the reactor, various noble metals including the catalyst according to the above second aspect or metal precursors may be dissolved in various polar solvents to prepare a noble metal or metal precursor solution. As for a carrier for the noble metal catalyst, carbon may be selected due to very high selectivity for oxygen, wherein carbon with a large specific surface area such as activated carbon, graphene, carbon nanotube, and the like may be used.

The carbon carrier may be washed by using a nitric acid solution and further three times or more washed with purified water and then, dried before use. The noble metal may be supported to secure a high dispersion degree by using an excess solution impregnation/immersion method.

For example, a platinum precursor solution is prepared and then, impregnated/immersed in a carbon carrier with a large specific surface area, washed, and dried.

For another example, in order to prepare a ternary oxide catalyst of Ru, Mn, and Co, an Mn precursor and a Co precursor solution are respectively prepared, surface-modified with an anionic crosslinking agent, impregnated/immersed in a Ru precursor (if necessary, a Ru precursor solution), washed, and dried.

Still for another example, in order to prepare a binary oxide catalyst of Au and Ce, a Ce precursor solution is prepared, surface-modified by using an anionic crosslinking agent, impregnated/immersed in an Au precursor solution, washed, and dried.

These washed and dried carbon carriers (e.g., activated carbon) or the carrier having the structure represented by Chemical Formula 9 is put in a round flask and then, mixed and supported by using a rotatory evaporation drier. After removing the purified water through evaporation, the carbon carriers or the carrier having the structure represented by Chemical Formula 9 is dried in a drier. The dried carbon carriers or carrier having the structure represented by Chemical Formula 9 may be reduced by heating at 400 °C to 500 °C in an electric furnace under a hydrogen atmosphere to obtain a noble metal-supported carbon body or a metal catalyst component-supported heterogeneous catalyst. Herein, the reduction temperature may be a temperature of a typical reduction reaction.

The prepared catalyst component-supported carbon body or catalyst having the structure represented by Chemical Formula 9 is filled in a vertically installed tubular reactor. Herein, when both the catalyst-free oxidation reactor and the oxidation reactor are present, the corresponding catalyst is filled in the tubular oxidation reactor.

After the filling, the reactor is maintained under vacuum at 100 °C to 500 °C to remove impurities absorbed therein.

The reactor (corresponding to a top where the catalyst is filled) is adjusted within a range of 1 to 10 bar by using the oxidizing agent such as oxygen gas and the like, because at 1 bar or less, the reaction rate may be deteriorated by adsorption of the oxygen on the catalyst surface but at 10 bar or more, insignificantly increase. Accordingly, the pressure is optimal within a range of 2 to 4 bar.

In addition, the reactor may be set at an oxidation reaction temperature of 40 °C to 200 °C. When 40 °C or less, the reaction rate may be slow, but when 200 °C or higher, the oxygen is less adsorbed on the catalyst surface, thereby deteriorating reactivity. An optimal temperature is within a range of 40°C to 180 °C.

The liquid mass feeder installed at the top of the reactor (the top of the filled catalyst) for an oxidation reaction may be used to supply the solution in which the aromatic heterocyclic compound or its modified compound is dissolved. The supplied solution in which the aromatic heterocyclic compound or its modified compound is dissolved flows to the bottom of the reactor by gravity and reacts with the catalyst to carry out the oxidation reaction.

The separation of the reaction products from the solvent may be performed by using a method generally used in a chemical product-manufacturing process. For example, after heating or cooling the reaction products, the solvent may be removed by distillation, or after producing crystals of the reaction products, the products or the product crystals may be recovered.

Accordingly, the method of the present invention may efficiently provide the desired reaction products by using an optimal raw material for providing desired products with a heterogeneous catalyst to perform a continuous reaction under the appropriate reaction conditions.

### DETAILED DESCRIPTION OF THE EMBODIMENTS

Hereinafter, the present invention will be described with reference to examples. However, the following examples are intended to illustrate the present invention, and the scope of the present invention is not limited to these only.

### <Examples>

Preparation Example 1: Preparation of Raw Material 1 having the structure represented by Chemical Formula 1

Among compounds having the structure represented by Chemical Formula 1 as raw materials, a compound with R1=aldehyde and R2=ChH₂Cl (corresponding to a compound having a structure represented by Chemical Formula 2) was prepared through conversion from wood.

Preparation Example 2: Preparation of Raw Material 2 having the structure represented by Chemical Formula 1

Among compounds having the structure represented by Chemical Formula 1 as raw materials, a compound with R1=R2=aldehyde (corresponding to 5-hydroxymethyl furfural that does not meet the proviso@@@of Chemical Formula 1) was prepared.

Preparation Example 3: Preparation of Heterogeneous Catalyst (4 wt% Ru-MnCo₂O₄)

An Mn precursor (Mn(CH₃COO)₂ and a Co precursor (Co(CH₄COO)₃) were taken in a weight ratio of 1:1 and respectively dissolved in a DIW solvent with a weight 50 times that of each precursor to prepare an Mn precursor solution and a Co precursor solution.

The prepared precursor solutions were mixed each other and then, mixed with a citric acid solution as an anionic crosslinking agent to prepare a porous central metal-organic framework material.

The porous central metal-organic framework material was activated to have a spherical shape to obtain spherical Ru-MnCo₂O₄ as a framework material in which binding sites were generated.

Subsequently, the MnCo₂O₄ was stirred with a Ru precursor (RuCl₃*3H₂O) as a metal catalyst component for 12 hrs under a N₂ atmosphere, and NaBH₄ was dropped thereto at 10 times as much as that of the Ru precursor and then, reacted by stirring at 500 rpm under an N₂ atmosphere at room temperature for 24 hours to obtain 4% Ru/MnCo₂O₄.

Preparation Example 4: Preparation of Heterogeneous Catalyst (2 wt% Au-CeO₂)

A Ce precursor (Ce(CH₃COO)₂) was taken and dissolved in 20 g of a H₂O solvent and 0.01 mole of an NaOH aqueous solution to prepare a Ce precursor solution. The prepared Ce precursor solution was stirred at room temperature under a nitrogen atmosphere for 12 hours to obtain a spherical CeO₂ support.

The CeO₂ support was stirred with an Au precursor (AuCl₃*3H₂O) as an inactive catalyst component for 12 hr under a N₂ atmosphere, and NaNH₄ was dropped thereto at 10 times as much as that of the Au precursor and then, reacted by stirring at 500 rpm under a N₂ atmosphere at room temperature for 24 hours to obtain a 2 wt% Au-CeO₂ catalyst.

Preparation Example 5: Preparation of Heterogeneous Catalyst (4 wt% Ru-MnCo₂O₄)

An Mn precursor (Mn(CH₃COO)₂ and a Co precursor (Co(CH₄COO)₃) were taken in a weight ratio of about 1:1 and dissolved in a DIW solvent at a weight of 50 times as much as that of each precursor to prepare an Mn precursor solution and a Co precursor solution.

The prepared precursor solutions were mixed and then, stirred at room temperature under a N₂ atmosphere for 12 hours to obtain a framework material.

Subsequently, the MnCo₂O₄ was stirred with a Ru precursor (RuCl₃*3H₂O) as a metal catalyst component for 12 hrs under a N₂ atmosphere, and NaBH₄ was dropped thereto at about 10 times as much as that of the Ru precursor and then, reacted by stirring at 500 rpm under a N₂ atmosphere at room temperature for 24 hours to obtain 4% Ru/MnCo₂O₄.

### <Example 1: Oxidation Reaction Experiment >

After connecting two types of columns including a reacting region, having a diameter of 20 cm and a height of 100 cm, and made of a SUS material to a reactor, the reacting region in the rear column, which was used as an oxidation reactor, was filled with 1.5 kg of Ru-MnCo₂O₄ as a heterogeneous catalyst, while using the front column as a catalyst-free oxidation reactor.

Herein, the Ru-MnCo₂O₄, which was prepared in Preparation Example 3, was filled. After the filling, impurities absorbed thereon were removed by maintaining the reactor at 150 °C.

In the storage tank of the raw material aromatic heterocyclic compound, 5 L of a solution prepared by dissolving the aromatic heterocyclic compound (the compound of Chemical Formula 1, wherein R1=aldehyde, R2=CH₂Cl, corresponding to the material prepared in Preparation Example 1) in acetonitrile at a concentration of 1 wt% as a polar solvent was filled. After the filling, oxygen gas was used for pressurization to 3 bar. Herein, since the applied pressure is proportional to an amount of the oxygen dissolved in the aromatic heterocyclic compound, a higher pressure may improve a reaction rate.

The front column was supplied with the raw material in a solution state pressurized by the oxygen from the top of the column by using a liquid mass feeder, and an internal pressure of the column was adjusted within a range of 1 to 5 bar. The supplied aromatic heterocyclic compound solution flew to the bottom of the column, wherein 15 g of tetrabutylammonium chloride (TBAC) as an onium-based compound and 10 g of NaOAc as an ion activator were added therewith to the front column.

Herein, when a portion of the reactant was extracted to check a structure, a compound turned out to have a structure represented by Chemical Formula 3.

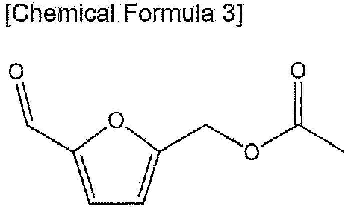

Subsequently, the reactant was transported to the rear column to contact with the catalyst therein, while NaHCO₃ was intermittently added to satisfy pH of the reaction solution within a range of 7 to11, to cause an oxidation reaction, from which 3 wt% NaHCOs (in DIW solution) was three times washed at three times as much as the reactant to obtain a crude composition including oxides.

### <Example 2: Oxidation Reaction Experiment >

An oxidation reaction was performed in the same manner as in Example 1 by using the same method as in Example 1, except that 4 wt% Ru-MnCo₂O₄ according to Preparation Example 3 and 2 wt% Au-CeO₂ according to Preparation Example 4 were used.

Specifically, used was a reactor in which two types of columns including a reacting region, having a diameter of 20 cm and a height of 100 cm, and made of a SUS material were connected, wherein the front column was used as a catalyst-free oxidation reactor, while the rear column was used as an oxidation reactor, and the reacting region of the rear column was filled with 1.5 kg of Au-CeO₂ as a heterogeneous catalyst.

Herein, the Au-CeO₂, which was prepared in Preparation Example 4, was filled. After the filling, impurities absorbed thereon were removed by maintaining the reactor at 150 °C under vacuum.

In the storage tank of the raw material aromatic heterocyclic compound, 5 L of a solution prepared by dissolving an aromatic heterocyclic compound (a compound of Chemical Formula 1, wherein R1=aldehyde, R2=CH₂Cl, corresponding to the material prepared in Preparation Example 1) in DMSO at a concentration of 1 wt% as a polar solvent was filled. After the filling, oxygen gas was used for the pressurization to 3 bar. Herein, since the applied pressure is proportional to an amount of the oxygen dissolved in the aromatic heterocyclic compound, a higher pressure may improve a reaction rate.

The front column was supplied with the raw material in a solution state pressurized by the oxygen from the top of the column by using a liquid mass feeder, and an internal pressure of the column was adjusted within a range of 1 to 5 bar. The supplied aromatic heterocyclic compound solution flew to the bottom of the column, wherein as a result of checking a structure by extracting a portion of the catalyst-free oxidation reactant, a compound with the structure represented by Chemical Formula 4 was confirmed.

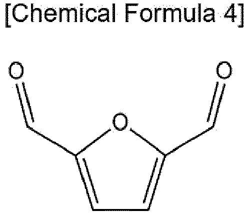

Subsequently, the solvent (DMSO) in the front column was substituted with tertiarybutyl hypochlorite and then, proceeded with a reaction at room temperature for 24 hours.

Additionally, as a result of checking a structure by extracting a portion of the reactant, a compound with the structure represented by Chemical Formula 4 was confirmed.

Subsequently, the obtained reactant was transported to the rear column to contact with the catalyst in the column, and NaHCO₃ was intermittently added thereto to satisfy the reaction solution within a range of pH 7 to 11 to cause an oxidation reaction, from which 3wt% NaHCOs in DIW solution was washed with water at three times as much as the reactant to obtain a crude composition including oxides.

### <Comparative Example 1: Oxidation Reaction Experiment

A crude composition including oxides was obtained by repeating the same process as in Example 1 except that the raw material of Preparation Example 1 was replaced with the raw material of Preparation Example 2.

### <Comparative Example 2: Oxidation Reaction Experiment>

A crude composition including oxides was obtained in the same manner as in Example 1 by using the same method as used in Example 1, except that 4 wt% Ru-MnCo₂O₄ of Preparation Example 5 was used instead of 1 wt% Ru-MnCo₂O₄ of Preparation Example 3.

Specifically, used was a reactor in which two types of columns including a reacting region, having a diameter of 20 cm and a height of 100 cm, and made of a SUS material were connected, wherein the front column was used as a catalyst-free oxidation reactor, while the rear column was used as an oxidation reactor, and the reacting region of the rear column was filled with 1.5 kg of Ru-MnCo₂O₄, a heterogenous catalyst.

Herein, the Ru-MnCo₂O₄, which was prepared in Preparation Example 5, was filled. After the filling, impurities absorbed thereon were removed by maintaining the reactor at 150 °C.

In the storage tank of the raw material aromatic heterocyclic compound, 5 L of a solution prepared by dissolving an aromatic heterocyclic compound (a compound of Chemical Formula 1, wherein R1=aldehyde, R2=CH₂Cl, corresponding to the material prepared in Preparation Example 1) in acetonitrile at a concentration of 1 wt% as a polar solvent was filled. After the filling, oxygen gas was used for the pressurization to 3 bar. Herein, since the applied pressure is proportional to an amount of the oxygen dissolved in the aromatic heterocyclic compound, a higher pressure may improve a reaction rate.

The front column was supplied with the raw material in a solution state pressurized by the oxygen from the top of the column by using a liquid mass feeder, and an internal pressure of the column was adjusted within a range of 1 to 5 bar. The supplied aromatic heterocyclic compound solution flew to the bottom of the column by gravity, wherein 15 g of tetrabutylammonium chloride (TBAC) as an onium-based compound and 10 g of NaOAc as an ion activator were added therewith to the front column.

Herein, when a portion of the reactant was extracted to check a structure, a compound turned out to have the structure represented by Chemical Formula 3.

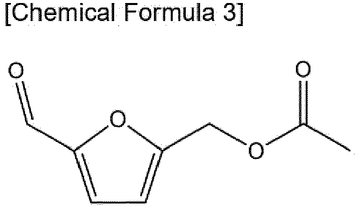

Subsequently, the reactant was transported to the rear column to contact with the catalyst in the column, while NaHCO₃ was intermittently added to satisfy the reaction solution within a range of pH 7 to11 to cause an oxidation reaction, from which 3 wt% NaHCOs (in DIW solution) was three times washed at three times as much as the reactant to obtain a crude composition including oxides.

### <Evaluation>

### 1) Yield Evaluation

Each content of the compound with the structure represented by Chemical Formula 5, the compound with the structure represented by Chemical Formula 6, and the compound with the structure represented by Chemical Formula 7 was measured through component analysis of the crude compositions synthesized in each method shown in Examples 1 to 2 and Comparative Examples 1 to 2, and the results are shown in Table 1.

**(Table 1)**

| | Example 1 | Example 2 | Comparative Example 1 | Comparative Example 2 |
|---|---|---|---|---|
| Chemical Formula 5 structure compound (wt%) | 99 | 98 | 65.5 | 73.5 |
| Chemical Formula 6 structure compound (wt%) | 0.3 | 0.4 | 30 | 23 |
| Chemical Formula 7 structure compound (wt%) | 0.7 | 1.6 | 4.5 | 3.5 |

As shown in Table 1, when the raw material of Preparation Example 1 according to the present invention was used, a yield of 98 wt% or more was confirmed for both of the heterogeneous catalysts of Preparation Examples 3 to 4.

On the contrary, when the raw material of Comparative Example 1 was used, an inferior yield of 65.5% was confirmed for each heterogeneous catalyst of Preparation Examples 3 to 5.

In addition, when the catalyst of Preparation Example 5 was used without using the appropriate crosslinking agent of Comparative Example 2, an inferior yield of 73.5% was also confirmed.

### 2) Purity Evaluation

The compound with the structure represented by Chemical Formula 5, which was confirmed in the yield evaluation, also was evaluated with respect to purity by using a sample obtained in Example 1 and a standard material in the related art, which is manufactured by Sigma Aldrich Co., Ltd. as a control example, and measuring with HPLC, and the purity comparison analysis results are shown in Table 2.

**(Table 2)**

| | Example 1 | Control |
|---|---|---|
| Purity | 97.5 | 95 |

As shown in Table 2, the corresponding compound excessively synthesized by using the raw material provided in the present invention turned out to exhibit high purity.

### 3) Evaluation of Conversion Rate

When the crude compositions according to Examples 1 to 2 were measured with respect to a conversion rate according to a temperature and a reaction pressure during the oxidation reaction, the compound having the structure represented by Chemical Formula 5 exhibited an excellent conversion rate within a range of 100 °C to 180 °C under a pressure of 1 to 4 atm.

### 4) Evaluation of Effect of Pressure

During the conversion rate evaluation of the 3), an effect of the pressure at the same temperature was evaluated. The corresponding pressure turned out to affect adsorption power of carbon and platinum onto oxygen or adsorption power of a central metal, a transition metal, and a metal catalyst component onto the oxygen and exhibited an excellent conversion rate at a pressure of 1 to 5 atm, particularly, 2 atm.

The features, structures, effects, etc. illustrated in each of the above-described embodiments can be combined or modified for other embodiments by those skilled in the art. Therefore, contents related to such combinations and modifications should be construed as being included in the scope of the present invention.

### [Industrial Applicability]

The present invention provides a method for preparing a dicarboxyl acid aromatic heterocyclic compound that is environmentally friendly and has improved production efficiency by using bio-based raw materials as the aromatic heterocyclic compound raw material.

## Claims

1. A heterogeneous catalyst, comprising
a catalyst for preparing dicarboxyl acid aromatic heterocyclic compound,
wherein a structure in which cations of a metal catalyst component are bonded to a porous central metal-organic framework material.

2. The heterogeneous catalyst of claim 1, wherein
the metal catalyst component is one or more selected from Fe, Ni, Ru, Rh, Pd, Os, Ir, Au, and Pt.

3. The heterogeneous catalyst of claim 1, wherein
the porous central metal is one or more types selected from a skeleton support material, an active metal, and an activity-promoting metal.

4. The heterogeneous catalyst of claim 1, wherein
the organic framework material is an anion provided from an anionic crosslinking agent.

5. The heterogeneous catalyst of claim 4, wherein
the anionic crosslinking agent is one or more polyanionic substances selected from citric acid, tartaric acid, malic acid, malonic acid, and tartaric acid.

6. The heterogeneous catalyst of claim 1, wherein
the heterogeneous catalyst produces a dicarboxyl acid aromatic heterocyclic compound by oxidizing an aromatic heterocyclic raw material having a structure represented by Chemical Formula 1: 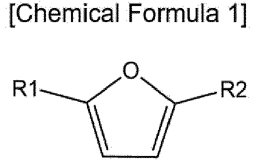 (wherein, in above chemical formula, any one of R1 and R2 is an aldehyde and the other is a substituted or unsubstituted (C1-C20) alkyl, acetoxy, or aldehyde, the substituted (C1-C20) alkyl is substituted with one or more functional groups selected from bromine, chlorine, fluorine, and iodine).

7. The heterogeneous catalyst of claim 1, wherein
the heterogeneous catalyst is prepared by oxidizing one or more compounds selected from a compound having a structure represented by Chemical Formula 2, a compound having a structure represented by Chemical Formula 3, and a compound having a structure represented by Chemical Formula 4 in a polar solvent to obtain an oxide having the structure represented by Chemical Formula 5:

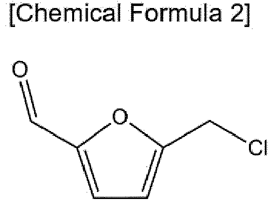

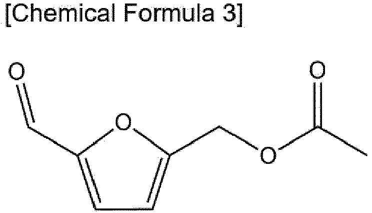

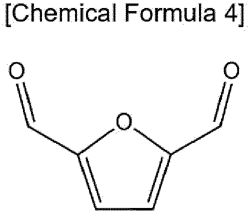

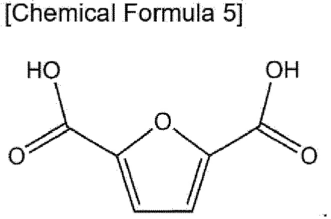

8. The heterogeneous catalyst of claim 6, wherein
the heterogeneous catalyst one or more selected from Ru/MnCo₂O₄, Au/CeO₂, Ru/C and PtIC at a molar ratio of 0.4 to 1.0 based on 1 mole of the aromatic heterocyclic compound having the structure represented by Chemical Formula 1.

9. A method for preparing a heterogeneous catalyst comprising
preparing a solution including a porous central metal oxide;
preparing a porous central metal-organic framework material by reacting the porous central metal oxide solution with an anionic crosslinking agent; and
impregnating the porous central metal-organic framework material with a precursor solution of the metal catalyst component to provide a structure in which cations of the metal catalyst component are bonded.

10. The method of claim 9, wherein
the second step includes
a 2-1 step of mixing the porous central metal oxide solution and an anionic crosslinking agent;
a 2-2 step of heating the mixed solution after the step 2-1 to a first temperature and then performing a primary reaction of the mixed solution; and
a 2-3 step of heating the mixed solution after the step 2-2 to a second temperature higher than the first temperature and then subjecting the mixed solution to a secondary reaction

11. The method of claim 9, wherein
the third step includes
a 3-1 step of impregnating the reactant after the second step with a precursor solution of the metal catalyst component obtained by dissolving the precursor of the metal catalyst component in a solvent; and
a 3-2 step of firing the impregnated product after step 3-1 at a third temperature lower than the second temperature.

12. The method of claim 10 or claim 11, wherein
the solvent used to prepare the porous central metal oxide solution and the precursor solution of the metal catalyst component is water, alcohol, or a combination thereof.

13. The method of claim 9, wherein
the anionic crosslinking agent is citric acid, tartaric acid, malic acid, malonic acid, tartaric acid, and a combination thereof.

14. The method of claim 10, wherein
the first temperature is in the range of 40 to 200 °C,
the second temperature is in the range of 400 to 1000 °C, and
the step 2-3 is performed for 3 to 12 hours.

15. The method of claim 11, wherein
the step 3-1 is performed in the range of 40 to 80 °C, and
the third temperature is in the range of 40 to 800 °C.

16. A method for preparing a dicarboxyl acid aromatic heterocyclic compound, comprising
subjecting the above-described aromatic heterocyclic compound raw material to an oxidation reaction in a polar solvent in the presence of a heterogeneous catalyst to prepare a dicarboxyl acid aromatic heterocyclic compound,
wherein the heterogeneous catalyst is the catalyst of any one of claim 1 to claim 8, and
the aromatic heterocyclic compound raw material is a hydroxyl-free compound having a structure represented by Chemical Formula 1: 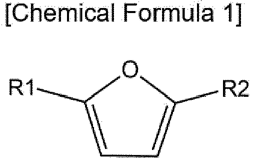 (wherein, in the above chemical formula, any one of R1 and R2 is an aldehyde, and the others are substituted or unsubstituted (C1 to C20) alkyl, acetoxy, or aldehyde, and the substituted (C1 to C20) alkyl is substituted with one or more functional groups selected from bromine, chlorine, fluorine and iodine).

17. The method of claim 16, wherein
the aromatic heterocyclic compound having the structure represented by Chemical Formula 1 is used in a state modified to the structure represented by Chemical Formula 3 or the structure represented by Chemical Formula 4:

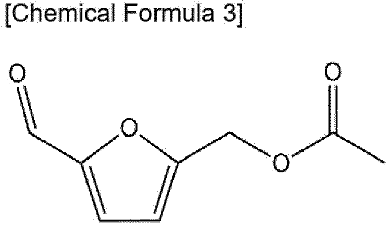

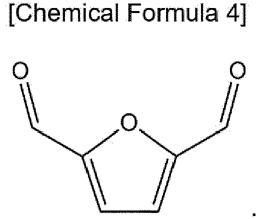

18. The method of claim 16, wherein
the oxidation reaction is performed by a catalyst-free oxidation step of oxidizing an aromatic heterocyclic compound having a structure represented by Chemical Formula 2 at a fourth temperature in a polar solvent in the presence of a halogen-based ammonium compound and an ion activator to produce an aromatic heterocyclic compound having a structure represented by Chemical Formula 3; and an oxidation step of oxidizing an aromatic heterocyclic compound having the structure represented by Chemical Formula 3 in a polar solvent in the presence of a basic material and a heterogeneous catalyst at a fifth temperature higher than the fourth temperature to produce a dicarboxyl acid aromatic heterocyclic compound:

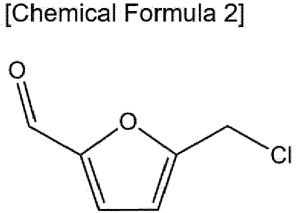

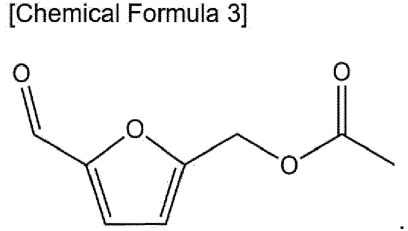

19. The method of claim 16, wherein
the oxidation reaction is performed by
a first catalyst-free oxidation step of oxidizing an aromatic heterocyclic compound raw material having a structure represented by Chemical Formula 2 at a sixth temperature in a polar solvent;
a second catalyst-free oxidation step of substituting the polar solvent with another type of polar solvent and performing an oxidation reaction at a seventh temperature lower than the sixth temperature to produce an aromatic heterocyclic compound having a structure represented by Chemical Formula 4; and
an oxidation step of oxidizing an aromatic heterocyclic compound having the structure represented by Chemical Formula 4 in a polar solvent at an eighth temperature lower than the sixth temperature in the presence of a heterogeneous catalyst to produce a dicarboxyl acid aromatic heterocyclic compound:
